# EUROPEAN PATENT APPLICATION

(11) **EP 3 875 088 A1**
(43) Date of publication of application: **08.09.2021**
(21) Application number: 19877846.6
(22) Date of filing: 31.10.2019
(51) Int. Cl.: A61K 31/505, A61J 3/00, A61K 9/14, A61K 9/48, A61K 47/02, A61P 35/02

(54) **PACKAGE FOR MEDICAL COMPOSITION CONTAINING ANTI-TUMOR AGENT**

(30) Priority: 31.10.2018 JP 2018204850
(71) Applicant: FUJIFILM Corporation, Tokyo 106-8620 (JP)
(72) Inventor: OURA Tai, Toyama-shi, Toyama 930-8508 (JP)
(74) Representative: Hoffmann Eitle
(86) International application number: PCT/JP2019/042749
(87) International publication number: WO 2020/090969

(57) **Abstract**

An object of the present invention is to provide a packaging body that can stably store a pharmaceutical composition containing a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide. According to the present invention, there is provide a packaging body obtained by packaging a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide, together with zeolite, and a method for stabilizing a pharmaceutical composition.

## Description

### Field of the Invention

The present invention relates to a packaging body that stably stores a pharmaceutical composition containing a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide (hereinafter, referred to as Compound A).

### Description of the Related Art

Compound A is a medically useful compound that has an FLT3 inhibitory action and has a strong effect on hematological cancer such as acute myeloid leukemia (Patent Document 1). In the medical field, there is a demand on a highly effective and stable pharmaceutical drug, which is expected to be applied clinically in a wide range of administration formulation forms such as an oral agent, an injection agent, an ointment, and a suppository.

On the other hand, with the advancement of in-home medical care and the promotion of self-medication, the maintenance of medication adherence is an important issue in drug therapy. The most commonly used formulation form in-home medical care is an oral agent (Non-Patent Document 1).

### Prior Art Documents

### Patent Documents

Patent Document 1: WO2015/056683A

### Non-Patent Documents

Non-Patent Document 1: Japanese Journal of Pharmaceutical Health Care and Sciences, Vol. 34, No. 3, pp. 289 to 296, 2008

### SUMMARY OF THE INVENTION

In a pharmaceutical composition containing a succinate salt of Compound A, which is produced by a general manufacturing method, the amounts of related substances significantly increase over time as compared with the succinate salt of Compound A, and thus the stability of the succinate thereof is significantly reduced. There is a strong demand on a packaging body that can stably store the pharmaceutical composition containing a succinate salt of Compound A.

Under such circumstances, as a result of intensive studies, the inventors of the present invention have found that even in the case of a pharmaceutical composition containing a pharmaceutical additive that contains almost no moisture and has no hygroscopicity, moisture is incorporated thereto, and stability is reduced similarly to a pharmaceutical composition in which a hygroscopic pharmaceutical additive is blended, and even in the case of a packaging body in which silica gel and an oxygen scavenger having a drying function are added, related substances increase, sufficient stability is not obtained, and stability is not improved only by removing the moisture.

Furthermore, the inventors of the present invention have found that a packaging body obtained by packaging a pharmaceutical composition containing a succinate salt of Compound A together with zeolite can suppress an increase in related substances.

The present invention provides a packaging body with which a pharmaceutical composition containing a succinate salt of Compound A can be stably stored, and a method for stabilizing the pharmaceutical composition.

The present invention provides the followings.
<1> A packaging body formed by packaging a pharmaceutical composition containing a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide, together with zeolite.
<2> A method for stabilizing a pharmaceutical composition, comprising packaging a pharmaceutical composition containing a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide, together with zeolite.
   The present invention further provides the followings.
<3> The packaging body according to <1>, in which the pharmaceutical composition is a pharmaceutical composition containing a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide and at least one or more kinds selected from the group consisting of celluloses, sugars, and sugar alcohol.
<4> The packaging body according to <1>, in which in the pharmaceutical composition according to <3>, the celluloses are microcrystalline cellulose, the sugars are lactose, and the sugar alcohol is erythritol or mannitol.
<5> The packaging body according to <1>, in which the pharmaceutical composition is the pharmaceutical composition according to <3> or <4>, which further contains a lubricant.
<6> The packaging body according to <1>, in which in the pharmaceutical composition according to <5>, the lubricant is magnesium stearate or sodium stearyl fumarate.
<7> The packaging body according to <1>, in which the pharmaceutical composition is the pharmaceutical composition according to any one of <3> to <6>, which is produced by a dry-type granulation method.
<8> The packaging body according to <1>, in which the pharmaceutical composition is the pharmaceutical composition according to any one of <3> to <7>, which is a hard capsule.
   The present invention further provides the followings.
<9> The method for stabilizing a pharmaceutical composition according to <2>, in which the pharmaceutical composition is a pharmaceutical composition containing a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide and at least one or more kinds selected from the group consisting of celluloses, sugars, and sugar alcohol.
<10> The method for stabilizing a pharmaceutical composition according to <2>, in which in the pharmaceutical composition according to <9>, the celluloses are microcrystalline cellulose, the sugars are lactose, and the sugar alcohol is erythritol or mannitol.
<11> The method for stabilizing a pharmaceutical composition according to <2>, in which the pharmaceutical composition is the pharmaceutical composition according to <9> or <10>, which further contains a lubricant.
<12> The method for stabilizing a pharmaceutical composition according to <2>, in which in the pharmaceutical composition according to <11>, the lubricant is magnesium stearate or sodium stearyl fumarate.
<13> The method for stabilizing a pharmaceutical composition according to <2>, in which the pharmaceutical composition is the pharmaceutical composition according to any one of <9> to <12>, which is produced by a dry-type granulation method.
<14> The method for stabilizing a pharmaceutical composition according to <2>, in which the pharmaceutical composition is the pharmaceutical composition according to any one of <9> to <13>, which is a hard capsule.

The packaging body according to an aspect of the present invention, which is obtained by packaging a pharmaceutical composition containing a succinate salt of Compound A together with zeolite, suppresses the increase in related substances and is useful as a packaging body of a pharmaceutical composition, which has excellent stability.

### DESCRIPTION OF THE PREFERRED EMBODIMENTS

The present invention will be described in detail below.

As used in the present specification, % means the mass percentage unless otherwise particularly specified. In the present invention, the numerical range indicated by using "to" indicates a range including numerical values described before and after "to" as a minimum value and a maximum value, respectively. In addition, in the present invention, in a case where there are a plurality of substances corresponding to each component in the composition, the amount of each component in the composition means the total amount of the plurality of substances present in the composition unless otherwise particularly specified.

A succinate salt of Compound A used in the present invention can be produced, for example, by the method disclosed in WO2015/056683A.

A packaging body according to the embodiment of the present invention is a packaging body in which a pharmaceutical composition containing Compound A is enclosed together with zeolite in the same packaging container.

The packaging body according to the embodiment of the present invention can be produced by enclosing the pharmaceutical composition containing Compound A and zeolite in the same packaging container.

The pharmaceutical composition containing Compound A may be directly enclosed in a packaging container or may be enclosed in a packaging container after another packaging. For example, after the pharmaceutical composition containing Compound A is processed into a formulation in a PTP packaging sheet, the formulation may be enclosed in a packaging container.

Zeolite may be directly enclosed in a packaging container or may be mounted on a part of the packaging container. For example, in a case where the packaging container is a bottle, a member may be provided on the back side of the cap, in which zeolite is mounted.

Zeolite may be used as it is, may be used after being packaged in a non-woven fabric or the like, may be used after being processed into a sheet shape, or may be used after being processed into a plate-shaped molded product or a tablet.

Examples of the zeolite which is used in the present invention include synthetic zeolite and natural zeolite, and preferred examples thereof include synthetic zeolite.

Examples of the crystal structure of zeolite include an A type, an X type, ferrierite, MCM-22, ZSM-5, mordenite, an L type, a Y type, and a beta type, and preferred examples thereof include an A type and an X type. Examples of the A type include Zeolum A-3, A-4, and A-5 (Tosoh Corporation), and examples of the X type include Zeolum F-9 (Tosoh Corporation).

Examples of the shape of zeolite include a bead, a pellet, and a powder.

The amount of zeolite to be used is not particularly limited; however, for example, in a case of a glass bottle having an internal volume of 100 mL, the amount of zeolite to be used may be 1 to 50 g, preferably 1 to 30 g, and more preferably 5 to 15 g.

The packaging container is not particularly limited as long as it can maintain the obturation property in a case where a pharmaceutical composition is enclosed; however, for example, it may be a container that is impermeable or hardly permeable to light, moisture, and oxygen.

Examples of the container that is impermeable to light, moisture, and oxygen include a glass bottle, an aluminum can, an aluminum package, a plastic bag a surface of which is coated with a metal, and a plastic bottle a surface of which is coated with a metal.

Examples of the container that is hardly permeable to light, moisture, and oxygen include a polyethylene bottle and a plastic packaging container.

Examples of the pharmaceutical composition containing Compound A which is used in the present invention include a pharmaceutical composition containing Compound A.

The pharmaceutical composition is not particularly limited; however, examples thereof include a tablet, a hard capsule, granules, fine granules, powders, a fast-disintegrating tablet, a formulation dissoluble at use, a dry syrup, and a powder formulation, preferred examples thereof include a hard capsule and a tablet, and more preferred examples thereof include a hard capsule.

Examples of the hard capsule include a capsule manufactured using gelatin, hypromellose, pullulan, or the like as a raw material, and preferred examples thereof include a capsule manufactured using hypromellose. Examples thereof include Vcaps Plus (Lonza Group AG) and Quali-V (Qualicaps Co., Ltd.). The size of the hard capsule is preferably No. 0 to No. 4 and more preferably No. 2 to No. 4.

An excipient can be added to the pharmaceutical composition containing Compound A which is used in the present invention, as necessary.

Examples of the excipient include sugar alcohol such as erythritol, mannitol, xylitol, lactitol, isomalt, and sorbitol; sugars such as sucrose, lactose, maltose, hydrated trehalose, and glucose; cyclodextrins such as α-cyclodextrin, β-cyclodextrin, γ-cyclodextrin, hydroxypropyl β-cyclodextrin, and sulfobutyl ether β-cyclodextrin sodium; celluloses such as microcrystalline cellulose; and starches such as corn starch, potato starch, and pregelatinized starch.

These excipients may be added alone or in a combination of two or more thereof.

Examples of the preferred excipient include sugars, sugar alcohol, and celluloses.

The sugars are preferably lactose, and the sugar alcohol is preferably mannitol or erythritol and more preferably erythritol. The celluloses are preferably microcrystalline cellulose.

The amount of the excipient added is not particularly limited, and an amount determined depending on the dosage form may be added.

In the pharmaceutical composition containing Compound A which is used in the present invention a pharmaceutically acceptable pharmaceutical aid can be used within the range in which the effects of the present invention are not impaired.

Examples of the pharmaceutical aid include a disintegrant, a binder, a lubricant, a sweetening agent, a coloring agent, a flavoring agent, a surfactant, a coating agent, and a plasticizer.

Examples of the disintegrant include carmellose, carmellose calcium, croscarmellose sodium, carboxymethyl starch sodium, crospovidone, low-substituted hydroxypropylcellulose, and partially pregelatinized starch. Preferred examples thereof include crospovidone.

Examples of the binder include hydroxypropyl cellulose, carmellose sodium, and methyl cellulose, hypromellose, and polyvinyl alcohol.

Examples of the lubricant include stearic acid, magnesium stearate, sodium stearyl fumarate, calcium stearate, talc, hydrated silicon dioxide, light anhydrated silicic acid, and sucrose esters of fatty acids, preferred examples thereof include magnesium stearate, sodium stearyl fumarate, and hydrogenated oil, more preferred examples thereof include magnesium stearate and sodium stearyl fumarate, still more preferred examples thereof include magnesium stearate.

Examples of the sweetening agent include aspartame, saccharin, stevia, thaumatin, and acesulfame potassium.

Examples of the colorant include titanium dioxide, red ferric oxide, yellow ferric oxide, black iron oxide, Food Red No. 102, Food Yellow No. 4, and Food Yellow No. 5.

Examples of the flavoring agent include essential oils such as orange oil, lemon oil, peppermint oil, and pine oil; extracts such as orange extract and peppermint extract; flavors such as cherry flavor, vanilla flavor, and fruit flavor; powdered fragrances such as apple micron, banana micron, peach micron, strawberry micron, and orange micron; vanillin; and ethyl vanillin.

Examples of the surfactant include sodium lauryl sulfate, sodium dioctyl sulfosuccinate, polysorbate, and polyoxyethylene-hardened castor oil.

Examples of the coating agent include hypromellose, an aminoalkyl methacrylate copolymer E, an aminoalkyl methacrylate copolymer RS, ethyl cellulose, cellulose acetate phthalate, hydroxypropyl methyl cellulose phthalate, a methacrylic acid copolymer L, a methacrylic acid copolymer LD, and a methacrylic acid copolymer S.

Examples of the plasticizer include triethyl citrate, macrogol, triacetin, and propylene glycol.

These additives may be used alone or in a combination of two or more thereof.

The blending amount is not particularly limited, and blending may be carried out appropriately so that the effect thereof is sufficiently exhibited according to each purpose.

The method for producing the pharmaceutical composition containing Compound A which is used in the present invention is not particularly limited; however, examples thereof include a method in which an excipient and/or a pharmaceutical aid is mixed with a succinate salt of Compound A and granulated, and the obtained granulated product is formulated by a conventional method.

For example, in a case where the pharmaceutical composition is a hard capsule or a tablet, after granulating a mixture of raw materials, a hard capsule may be filled with the obtained granulated product, or the obtained granulated product may be tableted.

More specific examples thereof include the following method.
(1) A succinate salt of Compound A and one or more additives among an excipient, a disintegrant, and a lubricant are added and mixed to produce a mixed powder, the obtained mixed powder is compressed by a dry-type granulation method, a compression-molded product is produced, and the obtained compression-molded product is crushed and sized to obtain a granulated product.
(2) An excipient and/or a pharmaceutical aid is added to the obtained granulated product and mixed to obtain a mixed powder.
(3-1) The obtained mixed powder is tableted to obtain an uncoated tablet. Further, the obtained uncoated tablet may be film-coated.
(3-2) A hard capsule is filled with the obtained granulated product to obtain a hard capsule.

Examples of the granulation method include a wet-type granulation method, a dry-type granulation method, and a melting granulation method, preferred examples thereof include a wet-type granulation method and a dry-type granulation method, and more preferred examples thereof include a dry-type granulation method.

Examples of the wet-type granulation method include a fluidized bed type granulation method, a wet-type crushing granulation method, a rotary granulation method, a spray-drying type granulation method, and an extrusion granulation method, preferred examples thereof include a fluidized bed type granulation method and the wet-type crushing granulation method, and more preferred examples thereof include a fluidized bed type granulation method.

Examples of the dry-type granulation method include a compacting method, a slugging method, and a briquetting method, and preferred examples thereof include a compacting method. Examples of the compacting method include a method of using a roller compactor. In a case where a roller compactor is used, the pressure at the time of production, which is different depending on the device type used, is preferably 3 to 12 MPa, for example, in a case where TF-LABO (manufactured by Freund Corporation) is used.

In a case of being stored at 60°C for one month, the packaging body according to the embodiment of the present invention is a packaging body in which the amounts of related substances produced is preferably 1.2% or less, more preferably 0.8% or less, still more preferably 0.6% or less, and most preferably 0.4% or less.

The measurement method for related substances may be carried out by a conventional method, and examples thereof include the following method with high performance liquid chromatography (hereinafter, referred to as HPLC).

### (1) Preparation of standard solution

About 25 mg of a succinate salt of Compound A is precisely weighed, added to a dissolving solvent prepared by mixing water, acetonitrile, and trifluoroacetic acid at a ratio of 800:200:1, and irradiated with ultrasonic waves for dissolution, and then the volume is exactly adjusted to 50 mL. 1 mL of this solution is exactly quantitated and added to the dissolving solvent, and the volume is precisely adjusted to 100 mL.

### (2) Preparation of sample solution

A sample corresponding to about 25 mg of a succinate salt of Compound A is precisely weighed, added to a dissolving solvent, irradiated with ultrasonic waves for dissolution, and then the volume is exactly adjusted to 50 mL (0.5 mg/ml).

### (3) Measurement

5 µL of the standard solution and 5 µL of a sample solution are precisely quantitated and injected into an HPLC apparatus, and measurement is carried out under the following conditions.

### <Measurement condition>

Detector: ultraviolet absorption spectrophotometer (measurement wavelength: 300 nm)
Column: Meteoric Core C18, 3.0 mmϕ x 150 mm, particle size: 2.7 µm
Column temperature: 40°C
Sample temperature: constant temperature around 5°C
Mobile phase A: water / trifluoroacetic acid = 1,000/1
Mobile phase B: acetonitrile / trifluoroacetic acid = 1,000/1
Feeding of mobile phase: the mixing ratio between the mobile phase A and the mobile phase B is changed as follows to control the concentration gradient.

**[Table 1]**

| Time after injection (minutes) | Mobile phase A (vol%) | Mobile phase B (vol%) |
|---|---|---|
| 0∼5 | 80 | 20 |
| 5∼65 | 80→765 | 20→35 |
| 65∼80 | 65→735 | 35→65 |

Flow rate: 0.45 mL/min

### <Calculation expression>

Amount (%) of each of related substances Qi = (WS/WT) x (Ai/AS) x 1/100 x 100
WS: weighed amount (mg) of succinate salt of Compound A
WT: weighed amount (mg) of sample
Ai: peak surface area of each of related substances in sample solution
AS: average peak surface area of standard solution
1/100: dilution correction coefficient

The concentration of the sample solution (0.5 mg/mL) is a concentration within the range in which linearity is ensured in the calibration curve of the solution concentration versus the area percentage in HPLC, and the amount (%) of each of related substances indicates a value equivalent to the area percentage of each of the related substances in the HPLC measurement. Therefore, the area of each peak is measured by the automatic integration method, and the amounts of related substances can be calculated by the area percentage method.

For example, only a sample solution of a related substance is prepared and injected into an HPLC apparatus, the area percentage of the related substance (retention time: 72 minutes) is measured by the automatic integration method, and the mass of the related substance is calculated by the area percentage method, and a value equivalent to the mass (%) of the related substance can be obtained.

### Examples

The usefulness of the present invention will be described in Examples and Comparative Examples; however, the present invention is not limited thereto.

In Examples and Comparative Examples, as a desktop V-type mixer, VM-2 manufactured by TSUTSUI Scientific Instruments Co., Ltd. was used;
as a dry-type granulator, TF-LABO manufactured by Freund Corporation was used;
as magnesium stearate, Parteck™ LUB MST manufactured by Merck KGaA was used; and
as a hard capsule, Vcaps Plus made by Lonza Group AG was used.

### Example 1

### (1) Production of hard capsule

15 g of a succinate salt of Compound A, 16.4 g of erythritol (Erythritol T fine powder, manufactured by Mitsubishi-Chemical Foods Corporation), and 0.32 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.2% of the total amount, and the resultant mixture was mixed with the desktop V-type mixer for 30 minutes. A No. 3 hard capsule was filled with 193.8 mg of the obtained mixed powder to obtain a hard capsule.

### (2) Manufacture of packaging body

15 pieces of the hard capsule obtained in (1) and one piece of zeolite (10 g of Zeolum F-9 (manufactured by Tosoh Corporation), a non-woven fabric packaging product) were placed in a glass bottle having an internal volume of 50 mL, and the glass bottle was capped to obtain a packaging body.

### Example 2

### (1) Production of hard capsule

15 g of a succinate salt of Compound A, 8.2 g of microcrystalline cellulose (CEOLUS KG-1000, manufactured by Asahi Kasei Corporation), and 0.46 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.2% of the total amount, and the resultant mixture was mixed with the desktop V-type mixer for 30 minutes. A No. 3 hard capsule was filled with 144.6 mg of the obtained mixed powder to obtain a hard capsule.

### (2) Manufacture of packaging body

15 pieces of the hard capsule obtained in (1) and one piece of zeolite (10 g of Zeolum F-9 (manufactured by Tosoh Corporation), a non-woven fabric packaging product) were placed in a glass bottle having an internal volume of 50 mL, and the glass bottle was capped to obtain a packaging body.

### Example 3

### (1) Production of hard capsule

20 g of a succinate salt of Compound A, 21.9 g of microcrystalline cellulose (KG-1000, manufactured by Asahi Kasei Corporation), and 1.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.2% of the total amount, and the resultant mixture was mixed with the desktop V-type mixer for 30 minutes. A No. 3 hard capsule was filled with 197.8 mg of the obtained mixed powder to obtain a hard capsule.

### (2) Manufacture of packaging body

15 pieces of the hard capsule obtained in (1) and one piece of zeolite (10 g of Zeolum F-9 (manufactured by Tosoh Corporation), a non-woven fabric packaging product) were placed in a glass bottle having an internal volume of 100 mL, and the glass bottle was capped to obtain a packaging body.

### Comparative Example 1

15 pieces of the hard capsule obtained in Example 1 (1) were placed in a glass bottle having an internal volume of 50 mL, and the glass bottle was capped to obtain a packaging body.

### Comparative Example 2

15 pieces of the hard capsule obtained in Example 2 (1) were placed in a glass bottle having an internal volume of 50 mL, and the glass bottle was capped to obtain a packaging body.

### Comparative Example 3

15 pieces of the hard capsule obtained in Example 3 (1) were placed in a glass bottle having an internal volume of 100 mL, and the glass bottle was capped to obtain a packaging body.

### Comparative Example 4

15 pieces of the hard capsule obtained in Example 3 (1) and silica gel (2 g of SB (manufactured by MARUTANI Chemical Plant & Engineering Corporation), a non-woven fabric packaging product) were placed in a glass bottle having an internal volume of 50 mL, and the glass bottle was capped to obtain a packaging body.

### Comparative Example 5

15 pieces of the hard capsule obtained in Example 3 (1) and one piece of an oxygen scavenger (PharmaKeep KD-20, manufactured by Mitsubishi Gas Chemical Company, Inc.) having a drying function were placed in a glass bottle having an internal volume of 50 mL, and the glass bottle was capped to obtain a packaging body.

### Test example 1

The packaging bodies obtained in Examples 1 to 3 and Comparative Examples 1 to 5 were stored at 60°C for 1 month. The amount of the related substance (retention time: 72 minutes) after storage was measured by HPLC. The HPLC measurement was carried out under the following conditions. The peak surface area was measured by the automatic integration method, and the amount of the related substance was calculated by the area percentage method.

### (Measurement condition)

Detector: ultraviolet absorption spectrophotometer (measurement wavelength: 300 nm)
Column: Meteoric Core C18, 3.0 mmϕ x 150 mm, particle size: 2.7 µm
Column temperature: 40°C
Mobile phase A: water / trifluoroacetic acid = 1,000/1
Mobile phase B: acetonitrile / trifluoroacetic acid = 1,000/1
Mixing ratio between mobile phase during feeding: the mixing ratio between the mobile phase A to the mobile phase B was set as shown in Table 2. The concentration gradient was set to a linear gradient.

**[Table 2]**

| Time after injection (minutes) | Mobile phase A (vol%) | Mobile phase B (vol%) |
|---|---|---|
| 0∼5 | 80 | 20 |
| 5∼65 | 80→65 | 20→35 |
| 65∼80 | 65→35 | 35→65 |

Injection volume: 5 µL (a sample solution prepared to be 0.5 mg/mL was injected)
Flow rate: 0.45 mL/min

The results are shown in Table 3, Table 4, and Table 5.

**[Table 3]**

| | Environment adjusting agent | Produced amount of related substance (%) |
|---|---|---|
| Example 1 | Zeolite | 0.1 |
| Comparative Example 1 | Absent | 2.3 |

**[Table 4]**

| | Environment adjusting agent | Produced amount of related substance (%) |
|---|---|---|
| Example 2 | Zeolite | 0.2 |
| Comparative Example 2 | Absent | 2.5 |

**[Table 5]**

| | Environment adjusting agent | Produced amount of related substance (%) |
|---|---|---|
| Example 3 | Zeolite | 0.3 |
| Comparative Example 3 | Absent | 4.1 |
| Comparative Example 4 | Silica gel | 1.3 |
| Comparative Example 5 | PharmaKeep KD-20 | 2.1 |

The packaging body packaged together with zeolite greatly suppressed the increase in related substances and had stability, as compared with the Comparative Examples.

The packaging body according to the embodiment of the present invention suppresses the increase in related substances and is useful as a packaging body of a pharmaceutical composition, which has excellent stability.

## Claims

1. A packaging body formed by packaging a pharmaceutical composition containing a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide, together with zeolite.

2. A method for stabilizing a pharmaceutical composition, comprising packaging a pharmaceutical composition containing a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide, together with zeolite.
